(19) Europäisches Patentamt
European Patent Office
Office européen des brevets

(11) **EP 3 250 031 B1**

(12) **EUROPEAN PATENT SPECIFICATION**

(45) Date of publication and mention
of the grant of the patent:
**14.11.2018 Bulletin 2018/46**

(21) Application number: **16717716.1**

(22) Date of filing: **27.01.2016**

(51) Int Cl.:
***A01N 25/10*** *(2006.01)*    ***A61L 2/08*** *(2006.01)*
***C14C 11/00*** *(2006.01)*

(86) International application number:
**PCT/IT2016/000020**

(87) International publication number:
**WO 2016/120900 (04.08.2016 Gazette 2016/31)**

(54) **METHOD FOR OBTAINING SELF-CLEANING AND SELF-SANITIZING SURFACES OF FINISHED LEATHER**

VERFAHREN ZUM ERHALT VON SELBSTREINIGENDEN UND SELBSTDESINFIZIERENDEN OBERFLÄCHEN VON LEDER

SYSTÈME PERMETTANT D'OBTENIR DES SURFACES AUTONETTOYANTES ET AUTO-DESINFECTANT DE CUIR FINI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2015 IT VI20150031**

(43) Date of publication of application:
**06.12.2017 Bulletin 2017/49**

(73) Proprietor: **Gruppo Mastrotto S.p.A.**
**36071 Arzignano (VI) (IT)**

(72) Inventors:
• **POZZA, Giorgio**
**36071 Arzignano (VI) (IT)**

• **VESCO, Silvia**
**36071 Arzignano (VI) (IT)**
• **TAGLIAFERRI, Vincenzo**
**36071 Arzignano (VI) (IT)**

(74) Representative: **Burchielli, Riccardo et al**
**Barzano & Zanardo Roma S.p.A.**
**Via Piemonte 26**
**00187 Roma (IT)**

(56) References cited:
**WO-A1-98/38340     CN-A- 102 702 465**
**DE-A1- 19 736 309**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention generally relates to a method for obtaining self-cleaning and self-sanitizing surfaces of finished leather.

More particularly, the invention relates to a self-cleaning and self-sanitizing treatment of a finished leather, which is able to obtain a fast degradation of the organic material deposited on the leather surface and a pulling action against the cellular walls of the bacteria of the surface, which are therefore rendered harmless; furthermore, since the treatment is not effective in the absence of a direct lighting, to prevent proliferation of micro-organisms in periods of darkness, bacteriostatic components, non-toxic to humans, are also placed in the formulation, which act independently from the lighting exposure, thus ensuring a more complete protection.

The tanning process of the leather consists of multiple stages having as ultimate goal the transformation of the animal skin, which is easily subjected to bacterial attack, in a stable material, resistant to rot; before carrying out a generic process of tanning, the raw hide is normally planted, calcined, shaved, de-calcined and macerated.

All of these procedures (the so-called "riviera" treatment) have the object of removing unwanted substances (dirt, grease, blood, interfibrillary proteins, hyaluronic acid, etc.) from the skin and opening, in a controlled manner, the dermal fibers.

Depending upon the tanning agent used, said de-calcinated and macerated skin is normally subjected to a specific treatment, before the tanning phase, in order to minimize the reactivity of the collagen-tanning agent system to facilitate the penetration said tanning agent through the entire thickness of the leather.

At present there are different types of skins' tanning, such as mineral tanning (almost exclusively with basic salts of trivalent chromium), aldehyde tanning, tanning with synthetic tannins ("Syntan") and tanning with plant tannins (exclusively for soles leather).

In any case, a common feature of the known tanning agents is that said agents are able to interact with the skin's collagen, thus remaining permanently incorporated in the collagen's structure.

Other drawbacks related to the known techniques are also constituted by the presence of organic dirt and bacteria, that are deposited on the surface of the finished leather, thus resulting in a contamination of the atmosphere in contact with the article.

Moreover, a method for obtaining self-cleaning and self-sanitizing surfaces of fimished leather having the technical features of the preamble of the appended claim 1 is also known for example from WO98/38340A1, DE19736309A1 and CN102702465A.

In this context it is included the solution according to the present invention, which proposes to employ a method and the related formulations having a strong interest in the industrial field, such as surface coatings that give the product particular properties, such as self-cleaning and/or the capability to break down or reduce bacterial contamination in the skin; said formulations can be applied on the surface of the article without compromising its aesthetic and/or substantial features.

These and other results are obtained according to the present invention by proposing a "self-cleaning" treatment, which produces a fast degradation of organic material that is deposited on the leather surface; it consists in a particular change of the finishing phase, which has the effect of accelerating the natural disappearance of dirt without altering the other finishing features, and it cannot be carried out onto non-finished leather (suede, nubuck).

The main responsible of the degradation phenomenon is the oxygen of the atmosphere, which, due to some special components incorporated in the finishing layer, is made more aggressive towards the contaminants, thus accelerating the destruction; the oxidizing action also takes place against the gaseous organic substances that come into contact with the treated surface, for example atmospheric pollutants, which are transformed into non-toxic chemical species (carbon dioxide, water, etc.).

In addition to the destruction of solid or liquid contaminated agents present on the skin, a sanitization of the atmosphere in contact with the article is also obtained; the necessary requirement for obtaining an effective treatment is constituted by the fact that the surface is exposed to light, which promotes the action of the active elements of the formulation.

In addition to the above mentioned effects, a removing action against the cellular walls of the bacteria present on the surface is also obtained, thus rendering harmless said bacteria; since the treatment is not effective in the absence of direct lighting, to prevent proliferation of micro-organisms during dark periods specific bacteriostatic components which are not toxic to humans are also inserted in the formulation, said components acting independently from exposure light and thus ensuring a more complete protection.

The object of the present invention is therefore to indicate a method for obtaining self-cleaning and self-sanitizing surfaces of finished leather, which allows to obtain the previously described technical results.

Another object of the invention is to indicate a method for obtaining self-cleaning and self-sanitizing surfaces of finished leather, which can be carried out in a highly reliable manner and at low costs.

A further object of the invention is to indicate a method for obtaining self-cleaning and self-sanitizing surfaces of finished leather, which is simple and safe and which has an easy standardization.

It is therefore object of the present invention a method for obtaining self-cleaning and self-sanitizing surfaces of finished leather according to the enclosed claim 1; other technical features of said method are also given in the dependent claims.

In an advantageous manner, the realization of self-cleaning surfaces is obtained by making a so-called "photo-catalytic" formulation, that is to say a formulation based on a dispersion of stabilized nanometric titanium dioxide (titania), within a couple between an organic binder and a inorganic co-binder.

The nanometric titania, which is photo-activated, produces powerful oxidizing species, capable of attacking the majority of organic contaminants and to destroy them, thus allowing high levels of cleanliness and hygiene on the treated surfaces; such a formulation, in the form of a dispersion in a liquid phase, is applied on the skin by using various deposition technologies, depending on the type of the additive agent which is used.

The realization of self-sanitizing surfaces is obtained through the dispersion of active inorganic nano-particles (silver ions and/or copper ions supported on zeolites) or organic biocides within binders phases based on organic or siloxane resins having an organic modification; biocides, in the presence of contaminants such as fungi and bacteria, exert their capabilities by sanitizing the surface and by preventing the proliferation of pathogenic organisms.

The invention will be described in the following with particular reference to some illustrative embodiments.

The properties of self-cleaning of the finished leather surface are conferred by employing titanium dioxide as a photo-catalytic compound, which is capable of attacking the organic contaminant agents.

The mechanism of action is based on the moisture contained in the air and on a fraction of the solar light (from 300 to 400 nm) to produce reactive oxygen species that react with organic molecules of the contaminant agents by means of peroxidation mechanisms; it follows the oxidative removal of said contaminant agents.

The features of the titanium dioxide to form hydroxyl radicals makes it also effective as an antibacterial; in fact, the radical species that arise in the presence of light react with the peptidoglycan, which constitutes the bacterial cellular wall, and cause the alteration of the chemical structure. The process goes further by altering the cellular homeostasis and by causing the death of the bacteria.

The advantage of this process resides in the inability of the micro-organism to develop resistance mechanisms; however, the phenomenon occurs only when the titanium dioxide, which is partially incorporated in the coating, is irradiated with light.

To overcome the inefficiency of the photo-catalysis in the absence of illumination it is possible to use co-formulants which also provide for using inorganic ions such as silver or copper ions supported on zeolites.

The mechanism of action of the above species is different than the mechanism previously described.

As the inorganic ions are directly responsible for the bactericidal effect, they are able to dope the enzyme complexes involved in cellualar respiration, that is to say the enzymes making the DNA duplication and therefore the bacterial reproduction.

Also in this case the phenomenon of resistance development does not occur since the ions act as "parasitic species" against the ions involved in cellular metabolism.

This type of formulation has the advantage of inducing a certain sanitization of environments, where there is a lesser spreading of potentially pathogenic bacteria, and this synergy causes, for example, a reduction of management and maintenance costs of the environments having a high flow of people.

On the basis of what is stated, the treatment developed on the leather is able to attack the contaminants present on the surface (for example, wine stains, oil, coffee, smoke, etc.) and simultaneously inhibit the growth of potentially pathogenic micro-organisms or of agents which are also harmful for the article (for example, mustiness that can compromise the quality of the article).

The treatment does not alter the aesthetic characteristics (color, brightness degree, etc.) and/or the surface features of the article and moreover the treated skins shows performances which are not lower than the performances of the original ones, especially as regards the surface characteristics, such as, for example, hardness, resistance to scratching, resistance to predefined bending and fatigue cycles and/or resistance to chemical products.

The treatment of the present invention uses siloxane, vinylic and acrylic resins modified with polyurethane and the most appropriate formulation has been used by adding an alkyl-silane type water repellent protective agent added with photo-catalystic titanium dioxide.

Moreover, nanoparticles containing silver ions as biocide agents were also added to the mixtures.

To improve the dispersion of the nanoparticles, a dispersing agent based on an aqueous solution of a modified polymer containing groups having high-affinity with inorganic pigments was also joined to the mixture.

Finally, a siloxane-polyether copolymer containing fumed silica and acting as anti-foaming agent was alsoa added; the degree of gloss was optimized by adding an organic matting agent.

Finally, the mixture was diluted with de-ionized water to obtain the desired concentration of the formulation.

The treatment is preferably performed in two steps, which provide for placing a first and a second coating of the above mentioned active mixtures on the surface of the finished leather, as described in the following.

In relation to the deposition of the first coating, the mixture which is applied to the leather is made from the formulation of the following table:

| Product | Supplier | Description/Function | Active substances | Concentration |
|---|---|---|---|---|
| PLUS 7081 | ILVE | Filmogenic compound based on polymers and fillers | Polyacrylates, organic and inorganic fillers | 91.32% |
| Leveling agent K3 | GSC | Flow additive; improves the flowing of the mixture | Surfactants and volatile substances | 0.46% |
| Cross-linking agent U | ILVE | Cross-linking agent; improves the mechanical properties of the polymeric coating | Blocked di-isocyanate (CAS 7417-99-4) | 2.28% |
| Tinuvin 123 DW | Ciba | Aqueous dispersion; improves the resistance to the UV-rays and to the oxidation | Derivatives of 2,2,6,6-tetra-metyl-piperidine | 2.28% |
| Tinuvin 400 DW | Ciba | Aqueous dispersion; UV-B rays absorber | 2-hydroxylphenyl s-triazine (HPT) | 3.20% |
| Irganox 245 DW | Basf | Antioxidant | Sterically hindered carbolic derived | 0.46% |

The mixture of the above table (first coating) is applied by spraying it with the following operative conditions:

| Specific amount | Air pressure | Drying temperature | Drying time |
|---|---|---|---|
| 2.0 g/ft$^2$ | 1.4 atm | 110°C | 60 sec |

A second coating having photo-active features (photo-catalyst coating) is applied to the leather with the mixture's formulation of the following table:

| Product | Supplier | Description/Function | Active agents | Concentration |
|---|---|---|---|---|
| Water | - | Solvent/dispersing agent | Water | 26.87% |
| Aquaderm Fluido H | Lanxess | Water based auxiliary flow additive | Polyether-siloxane copolymer | 1.58% |
| Bindex9220 | GSC | Silicone emulsion for touch improvement | Silicone | 1.18% |
| Aquaderm additive agent GF | Lanxess | Silicone emulsion for touch improvement | Silicone | 1.18% |
| Astacin Novomatt GG | Basf | Polyurethane aqueous dispersion matting | Polyurethane | 47.28% |
| Astacin Matting LV TF | Basf | Polyurethane aqueous dispersion matting | Aliphatic polyurethane and inorganic opacifiers | 11.82% |
| Hardener 14 | Chime | Solvent aliphatic prepolymer for improving mechanical properties | Poly-hexamethylene diisocyanate | 6.30% |
| Aerodisp W 2730 X | Evonik | Photo-catalyst | Titanium dioxide | 1.58% |

(continued)

| Product | Supplier | Description/Function | Active agents | Concentration |
|---|---|---|---|---|
| Tego Phobe 1650 (alias VP-B 165) | Evonik | Hydrophobic agent | Silicone resin | 0.59% |
| Tego Foamex 810 | Evonik | Defoamer (anti-foam agent) | Polyether-siloxane copolymer with fumed silica | 0.49% |
| Sanitized BC A 21-41 | Sanitized | Biocide agent; micro powder with average size of the particles equal to 2 micron | Ceramic glass doped with silver ions | 0.59% |
| Tego Dispers 752 W | Evonik | Wetting and dispersing agent | Modified copolymer containing groups having high affinity with inorganic pigments | 0.54% |

The photo-catalyst mixture of the previous table (second coating) is applied with two subsequent steps of spraying with an intermediate drying, by using the following operating parameters:

| N. | Specific amount | Air pressure | Drying temperature | Drying time |
|---|---|---|---|---|
| 1 | 0.8 g/ft$^2$ | 1.2 atm | 100°C | 60 sec |
| 2 | 0.7 g/ft$^2$ | 1.2 atm | 100°C | 60 sec |

Practically, the resins were diluted in water while keeping the system under magnetic stirring for 30 minutes and, at the end, the mixtures were deposited on the surface of the leather by means of a spray gun with a nozzle diameter of 1.2 mm and with an outlet pressure of about 4 bar. The mixtures having the functionalized nanoparticles and the stabilized titanium dioxide were prepared by mixing the silver nanopowders, under mechanical stirring, with the dispersant and the water repellent for 30 minutes to obtain a homogeneous suspension and by adding, at the end, the resin, the matting agent, the anti-foam agent and the water.

The mixture was then allowed to stir for 15 minutes and, at the end of mixing, was filtered to remove any agglomerates and then applied by spraying it.

After said spraying phase a controlled drying phase (at 20°C, with humidity of 40%) can be provided, so as to reduce the times of out-dust of the article.

The evaluation of the photo-catalytic activity of the coating was then assessed by colorimetric measurements and with five different types of stains:

- methylene blue;
- red wine;
- coffee;
- EMPA 104 standard fouling fabric;
- EMPA 128 standard fouling fabric.

The treatment efficacy was assessed by comparing the behavior of samples subjected to treatment with untreated comparable samples and the application of the contaminant agents was carried out by using a standard cotton cloth (according to the ISO 105-F09 rules, having dimensions of 70 x 70 mm) which is placed in contact with the finished surface of the leather sample.

A defined quantity of contaminant agent (2 ml for liquids and 3 g for solids, according to the EN 15973 rule) was applied to the cloth and uniformly distributed on the whole surface and the sample thus prepared was left in the dark for 4 hours at a temperature of 23°C and a relative humidity of 50%; then the cloth was removed and the sample maintained for another 24 hours at the same conditions.

Excess of the contaminant agent was then removed with a damp cloth and the sample was exposed to light irradiation with a water cooled xenon light (the light exposure conditions used for the test are described in section 6.2 of the ISO standard rule 105-B02:1994).

For the purposes of benchmarking the specimen was half covered using the blind part of the metallic hold-samples element, so as to separate the degradation of the contaminant agent due to irradiation light from the degradation which is thermally induced; the photo-degradation of the individual specimen was then calculated by considering the variation ($\Delta E$) in the magnitude of the vector colorimetric E between the exposed area and the covered area.

The effectiveness of the photo-catalytic activity is the difference $\delta$ between said recorded $\Delta E$ of the treated sample, $\Delta E_t$, compared to the photo-catalytic activity of the untreated sample $\Delta E_{nt}$, namely:

$$\delta = \Delta E_t - \Delta E_{nt}.$$

Based on what has been said above, $\delta$ is a positive number, the value of which is quantitatively correlated to the photo-catalytic action and the trend of $\delta$ over time also allows to verify the dynamics of the degradation of the surface contaminant agents, taking into account the fact that the exposure duration is defined depending on the type of the contaminant agent which is used (the methylene blue, for example, is able to highlight the photo-catalytic effect already after an exposure of a few tens of minutes because of its poor light fastness, while the other contaminant agents require longer times).

The following table shows some values of $\delta$ obtained from the measurement with the standard D65 illuminant for the considered contaminant agents (between treated and untreated samples after contamination and exposure to light):

| Contaminant agent | $\Delta E_t$ | $\Delta E_{nt}$ | $\delta = \Delta E_t - \Delta E_{nt}$ |
|---|---|---|---|
| Methylene blue after 90 min | 33.92 | 16.96 | 16.96 |
| Red wine after 24 hours | 8.45 | 5.16 | 3.29 |
| Coffee after 48 hours | 8.58 | 6.18 | 2.40 |
| EMPA 104 after 48 hours | 3.45 | 0.63 | 2.82 |
| EMPA 128 after 48 hours | 8.08 | 5.32 | 2.76 |

Finally, in all the above cases the degradative effect induced by the photo-catalysis is confirmed by colorimetric measurements.

The biocidal activity of the coatings applied on the leather surfaces was evaluated according to the JIS Z 2801:2010 rule; the bacterial strain employed in the test is Escherichia coli ATCC 25922.

After sterilizing by UV irradiation a leather square of 20X40 mm coated with the above described functionalized formulation, said formulation has been inoculated onto a plate according to the standard rules.

The film containing one of the bacterial strains and deposited on the coated surface of the skin was analyzed at the beginning and 24 hours after said step of inoculation, taking care to block any traces of biocidal principles.

The dilutions of three independent inoculations have allowed us to evaluate the effectiveness of the leather article on which the functionalized coating was placed.

The killing of bacteria was evaluated by following equations:

$$\frac{L_{max} - L_{min}}{L_{mean}} \qquad (1)$$

$$R = (U_t - U_0) - (A_t - U_0) = U_t - A_t \qquad (2)$$

where:

$L_{max}$: maximum logarithmic number of viable bacteria,

$L_{min}$: minimum logarithmic number of viable bacteria,

$L_{mean}$: average of logarithmic numbers of viable bacteria of three samples,

R = antibacterial activity (Log Reduction),

$U_0$ = average of logarithmic numbers of viable bacteria immediately after inoculation onto untreated samples,

$U_t$ = average of logarithmic numbers of viable bacteria after inoculation onto untreated samples after 24 hours,

$A_t$ = average of logarithmic numbers of viable bacteria after inoculation onto bacterial samples after 24 hours.

The detail of the microbiological test is shown in the following table:

| Description | Value |
|---|---|
| Culture bacterial charge | $3.2 \times 10^5$/ml |
| Inoculated culture volume | 0.4 ml |
| Initial bacterial charge concentration/cm$^2$ | $1.1 \times 10^4$ ufc/cm$^2$ |
| Inoculated bacterial charge at $T_0$/cm$^2$ (first test) - $L_{max}$ | 12375 ufc/cm$^2$ (L = 4.09) |
| Inoculated bacterial charge at $T_0$/cm$^2$ (second test) | 11687.5 ufc/cm$^2$ (L = 4.07) |
| Inoculated bacterial charge at $T_0$/cm$^2$ (third test) - $L_{min}$ | 9125 ufc/cm$^2$ (L = 3.96) |
| Average inoculated bacterial charge at $T_0$/cm$^2$ - $L_{mean}$ ($U_0$) | 11062.5 ufc/cm$^2$ (L = 4.04) |
| 1° test: Lmax-Lmin/Lmedia<=0,2 | 0.03 (verified condition) |
| 2° test: bacterial charge/cm$^2$ > 6,2 103 - < 2,5 10$^4$ | $1.1 \times 10^4$ ufc/cm$^2$ (ver. cond.) |
| 3° test: bacterial charge after 24 hours onto an untreated sample > 6.2 x 10$^2$ /cm$^2$ ($U_t$) | $7.5 \times 10^6$ ufc/cm$^2$ (L = 6.88) (verified condition) |
| Bacterial charge after 24 hours onto a treated sample / cm$^2$ ($A_t$) | Absent (L < 0.63) |
| Value R ($U_t$-$A_t$) | 6.25 |

Because the test is passed if the Log Reduction is equal to or greater than 2, in this case it can be said that the treated surface shows a high biocidal activity.

The photo-catalytic activity due to the treatment is also obtained against the volatile substances of the air flowing around the surface of the leather. In particular, there is a reduction of the substances, organic or inorganic, susceptible to oxidation, which are converted into innocuous species (carbon dioxide, water, sulfate, nitrate, etc.).

The effect is experimentally measurable by using a standardized mixture of nitrogen oxides (NO + $NO_2$ = $NO_x$) in the air, the concentration of which is monitored with a chemiluminescence technique, by means of a suitable analyzer.

The samples having an area of 10 x 2 cm$^2$ were placed inside a quartz tubular reactor, inside of which were introduced 3 liters of standard gaseous mixture in which the initial concentration of nitrogen oxides corresponds to 600 ppb.

For activating the photo-catalysis process it is employed a light source with a power of 300 W and power density of about 15 W/m$^2$, placed at 25 cm from the surface of the test samples; the measurement of the concentration of $NO_2$ was overlooked as said substance can be adsorbed on the support even in the dark, while, on the contrary, the variation of the concentration of NO was measured, because it is substantially insensitive to the phenomena of adsorption of the support in the dark.

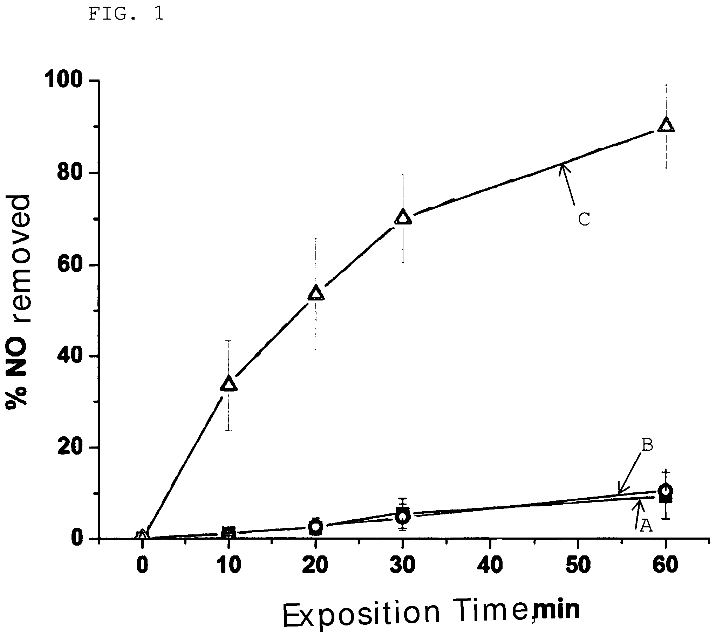

The trends of breakdown of the nitrogen oxides by photo-catalysis of the titanium dioxide are shown in detail in the enclosed figure 1 (in different scenarios which have been previously examined), which is a Cartesian graph of the percentage of removed nitrogen oxide (NO) as a function of the exposure time (T), measured in minutes.

The tests, replicated 4 times, were performed on three types of substrate:

- untreated sample of leather (curve A);
- sample of leather coated with the same binder part used for making the photo-catalytic coating, but without titanium dioxide (curve B);
- sample of leather coated with the photo-catalyst mixture (curve C).

The trends of breakdown of the nitrogen oxides show a remarkable rate of degradation of said oxides in case the sample has the photo-catalytic coating (curve C) and, as regards the reference formulation devoid of the photo-catalytic principle (curve B) and the sample as such (curve A), we are able to note a mild reduction of the concentration of the nitrogen oxides, as well as expected according to what is known from the scientific literature.

From the above description the technical features of the method for obtaining self-cleaning and self-sanitizing surfaces of finished leather are very clear, as well as the related advantages.

It should be noted, finally, that although the present invention has been described for illustrative but not limitative purposes, according to the preferred embodiments, other variations and/or modifications may be made by the man skilled in the art without departing from the relevant scope of the invention as defined by the appended claims.

**Claims**

1. Method for obtaining self-cleaning and self-sanitizing surfaces of finished leather, wherein said method comprises a first step of depositing a first coating layer of a first active mixture and a second step, subsequent to said first step, of depositing a second coating layer of a second active mixture, above said first layer, on the surface of said finished leather, wherein said first active mixture comprises a film-forming compound formed by polymer and filler, a flow additive, a cross-linking compound, an anti-oxidant compound and an aqueous dispersion, while said second active mixture comprises an auxiliary water-based flow additive compound, a silicone emulsion, a solvent aliphatic pre-polymer, a water repellent agent and solvent agents and/or wetting agents and/or dispersant agents and/or anti-foaming agents, **characterized in that** said second active mixture also comprises a photo-catalyst compound and a powder formulation with a biocide action, wherein, with reference to said first active mixture, said film-forming compound includes poly-acrylates and organic and inorganic fillers, said flow compound includes additive surfactants and volatile substances, said cross-linker compound includes blocked di-isocyanate and said anti-oxidant compound includes a sterically hindered phenol derivative and said aqueous dispersion includes derivatives of 2,2,6,6-tetramethylpiperidine and 2-hydroxyphenyl-s-triazine (HPT), while, with reference to said second active mixture, said auxiliary water-based flow additive compound includes a polyether-siloxane copolymer containing fumed silica, said silicone emulsion includes silicon, said solvent aliphatic pre-polymer includes polyhexamethyldiisocyanate said photo-catalyst compound includes titanium dioxide, said water-repellent agent includes a silicone resin, said powder formulation with a biocide action includes glass ceramic powder doped with inorganic ions, such as silver ions or copper ions, supported on zeolites and said solvent agents and/or said wetting agents and/or said dispersant agents and/or said anti-foaming agents include de-ionized water, aqueous dispersion of polyurethane, aliphatic polyurethane and/or inorganic matting agents, siloxane resins with modified polyurethane and/or vinyl resins and/or acrylic resins.

2. Method according to claim 1, **characterized in that** said first coating layer is applied by spraying at a pressure greater than the atmospheric pressure and, thereafter, is subjected to drying at a given temperature and for a specified time.

3. Method according to claim 1, **characterized in that** said second coating layer is applied by means of two phases of spraying at a pressure greater than the atmospheric pressure and by means of a drying phase, which is carried out between said spraying phases and which is performed at a given temperature and for a specified time.

4. Method according to claim 1, **characterized in that** said second active mixture includes a dispersing agent based on an aqueous solution of a modified polymer containing groups with high affinity to inorganic pigments.

5. Method according to claim 1, **characterized in that** said resins are diluted in water, kept under magnetic stirring for a specified time and then deposited on the surface of the leather by means of a spray gun and with a pressure which is substantially greater than the atmospheric pressure.

6. Method according to claim 1, **characterized in that** said active mixtures are prepared by mixing the powders of inorganic ions with said dispersing agents and/or said water-repellent agents for a given time to obtain a homogeneous suspension, by adding said resins, said matting agents, said anti-foaming agents and said de-ionized water, by providing a mechanical stirring of the solution for a given time and also by subjecting said solution to a filtration step to remove any agglomerates.

**Patentansprüche**

1. Verfahren zum Erhalten von selbstreinigenden und sich selbst sterilisierenden Oberflächen aus Fertigleder, wobei das Verfahren einen ersten Schritt des Aufbringens einer ersten Beschichtungsschicht aus einem ersten aktiven Gemisch und einen zweiten Schritt, der nach dem ersten Schritt erfolgt, des Abscheidens einer zweiten Beschichtungsschicht aus einem zweiten aktiven Gemisch auf der ersten Schicht auf der Oberfläche des Fertigleders, wobei das erste aktive Gemisch eine filmbildende Verbindung, die aus einem Polymer und einem Füllstoff gebildet ist, ein Fließhilfsmittel, eine Vernetzungsverbindung, eine Antioxidationsmittel-Verbindung und eine wässrige Dispersion umfasst, wohingegen das zweite aktive Gemisch eine zusätzliche Fließhilsmittelverbindung auf Wasserbasis, eine Silikonemulsion, ein aliphatisches Lösungsmittel-Präpolymer, ein Hydrophobierungsmittel und Lösungsmittel und/oder Benetzungsmittel und/oder Dispergiermittel und/oder Antischaummittel umfasst, **dadurch gekennzeichnet, dass** das zweite aktive Gemisch auch eine Photokatalysator-Verbindung und eine Pulverformulierung mit einer Biozid-Wirkung umfasst, wobei mit Bezug auf das erste aktive Gemisch die filmbildende Verbindung Polyacrylate und organische und anorganische Füllstoffe enthält, die Fließverbindung Zusatztenside und flüchtige Substanzen enthält, die Vernetzungsverbindung blockiertes Diisocyanat enthält und die Antioxidationsmittel-Verbindung ein sterisch gehindertes Phenolderivat enthält und die wässrige Dispersion Derivate von 2,2,6,6-Tetramethylpiperidin und 2-Hydroxyphenyl-s-triazin (HPT) enthält, wohingegen mit Bezug auf das zweite aktive Gemisch die zusätzliche Fließhilfsmittelverbindung auf Wasserbasis ein pyrogenes Siliciumdioxid enthaltendes Polyethersiloxan-Copolymer enthält, die Silikonemulsion Silicium enthält, das aliphatische Lösungsmittel-Präpolymer Polyhexamethyldiisocyanat enthält, die Photokatalysator-Verbindung Titandioxid enthält, das Hydrophobierungsmittel ein Silikonharz enthält, die Pulverformulierung mit einer Biozid-Wirkung Glaskeramikpulver, das mit anorganischen Ionen, wie etwa Silberionen oder Kupferionen, dotiert ist und auf Zeolithen trägergestützt ist, enthält und die Lösungsmittel und/oder die Benetzungsmittel und/oder die Dispergiermittel und/oder die Antischaummittel entionisiertes Wasser, eine wässrige Polyurethandispersion, aliphatisches Polyurethan und/oder anorganische Mattierungsmittel, Siloxanharze mit modifiziertem Polyurethan und/oder Vinylharze und/oder Acrylharze enthalten.

2. Verfahren nach Anspruch 1, **dadurch gekennzeichnet, dass** die erste Beschichtungsschicht durch Sprühen mit einem Druck, der größer als der Atmosphärendruck ist, aufgetragen wird und danach bei einer bestimmten Temperatur und für eine spezifische Zeit lang trocknen gelassen wird.

3. Verfahren nach Anspruch 1, **dadurch gekennzeichnet, dass** die zweite Beschichtungsschicht mittels zweier Sprühphasen mit einem Druck, der größer als der Atmosphärendruck ist, und mittels einer Trocknungsphase, die zwischen den Sprühphasen ausgeführt wird und die bei einer bestimmten Temperatur und für eine spezifische Zeit lang durchgeführt wird, aufgetragen wird.

4. Verfahren nach Anspruch 1, **dadurch gekennzeichnet, dass** das zweite aktive Gemisch ein Dispergiermittel enthält, das auf einer wässrigen Lösung eines modifizierten Polymers basiert, welches Gruppen mit hoher Affinität zu anorganischen Pigmenten enthält.

5. Verfahren nach Anspruch 1, **dadurch gekennzeichnet, dass** die Harze in Wasser verdünnt werden, für eine spezifische Zeit lang in einem Magnetrührer gerührt werden und dann auf die Oberfläche des Leders mittels einer Sprühpistole und mit einem Druck, der im Wesentlichen größer als der Atmosphärendruck ist, aufgebracht werden.

6. Verfahren nach Anspruch 1, **dadurch gekennzeichnet, dass** die aktiven Gemische hergestellt werden, indem die Pulver aus anorganischen Ionen mit den Dispergiermitteln und/oder den Hydrophobierungsmitteln für eine bestimmte Zeit lang vermengt werden, um eine homogene Suspension zu erhalten, die Harze, die Mattierungsmittel, die Antischaummittel und das entionisierte Wasser hinzugegeben werden, die Lösung für eine bestimmte Zeit lang mechanisch gerührt wird und die Lösung ebenfalls einem Filtrationsschritt unterzogen wird, um jegliche Agglomerate zu entfernen.

## Revendications

1. Procédé d'obtention de surfaces autonettoyantes et auto-assainissantes de cuir fini, dans lequel ledit procédé comprend une première étape de dépôt d'une première couche de revêtement d'un premier mélange actif et une seconde étape, subséquente à ladite première étape, de dépôt d'une seconde couche de revêtement d'un second mélange actif, au-dessus de ladite première couche, sur la surface dudit cuir fini, dans lequel ledit premier mélange actif comprend un composé filmogène formé d'un polymère et d'une charge, un additif d'écoulement, un composé de réticulation, un composé antioxydant et une dispersion aqueuse, tandis que le second mélange actif comprend un composé d'additif d'écoulement auxiliaire à base d'eau, une émulsion de silicone, un prépolymère de solvant aliphatique, un agent hydrofuge et des agents solvants et/ou des agents mouillants et/ou des agents dispersants et/ou des agents antimousse, **caractérisé en ce que** ledit second mélange actif comprend également un composé photocatalyseur, et une formulation de poudre à action biocide, dans lequel, en référence audit premier mélange actif, ledit composé filmogène inclut des polyacrylates et des charges organiques et inorganiques, ledit composé d'écoulement inclut des dispersants supplémentaires et des substances volatiles, ledit composé de réticulation inclut un diisocyanate bloqué et ledit composé antioxydant inclut un dérivé phénolique à empêchement stérique et ladite dispersion aqueuse inclut des dérivés de 2,2,6,6-tétraméthylpipéridine et de 2-hydroxyphényl-s-triazine (HPT), tandis que, en référence audit second mélange actif, le composé d'additif d'écoulement auxiliaire à base d'eau inclut un copolymère de polyéther-siloxane contenant de la silice sublimée, ladite émulsion de silicone inclut du silicium, ledit prépolymère de solvant aliphatique inclut du polyhexaméthyldiisocyanate, ledit composé photocatalyseur inclut du dioxyde de titane, l'agent hydrofuge inclut une résine de silicone, ladite formulation de poudre à action biocide inclut une poudre vitrocéramique dopée par des ions inorganiques, tels que des ions argent ou des ions cuivre, sur des supports de zéolithes et lesdits agents solvants et/ou lesdits agents mouillants et/ou lesdits agents dispersants et/ou lesdits agents antimousse incluent de l'eau désionisée, une dispersion aqueuse de polyuréthane, un polyuréthane aliphatique et/ou des agents de matité inorganiques, des résines de siloxane avec du polyuréthane modifié et/ou des résines vinyliques et/ou des résines acryliques.

2. Procédé selon la revendication 1, **caractérisé en ce que** ladite première couche de revêtement est appliquée par une pulvérisation à une pression supérieure à la pression atmosphérique et est ensuite soumise à un séchage à une température donnée et pendant une durée spécifiée.

3. Procédé selon la revendication 1, **caractérisé en ce que** ladite seconde couche de revêtement est appliquée au moyen de deux phases de pulvérisation à une pression supérieure à la pression atmosphérique et au moyen d'une phase de séchage qui est effectuée entre lesdites phases de pulvérisation et qui est effectuée à une température donnée et pendant une durée spécifiée.

4. Procédé selon la revendication 1, **caractérisé en ce que** ledit second mélange actif inclut un agent de dispersion à base d'une solution aqueuse d'un polymère modifé contenant des groupes ayant de grandes affinités pour des pigments inorganiques.

5. Procédé selon la revendication 1, **caractérisé en ce que** lesdites résines sont diluées dans l'eau, sont maintenues sous agitation magnétique pendant une durée spécifiée et sont ensuite déposées sur la surface du cuir au moyen d'un pistolet de pulvérisation et avec une pression qui est sensiblement supérieure à la pression atmosphérique.

6. Procédé selon la revendication 1, **caractérisé en ce que** lesdits mélanges actifs sont préparés par un mélange des poudres d'ions inorganiques avec lesdits agents de dispersion et/ou lesdits agents hydrofuges pendant une durée donnée pour obtenir une suspension homogène, par un ajout desdites résines, desdits agents de matité, desdits agents antimousse et de ladite eau désionisée, par une fourniture d'une agitation mécanique à la solution pendant une durée donnée et en soumettant également ladite solution à une étape de filtration pour éliminer les agglomérats éventuels.

FIG. 1

**EP 3 250 031 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9838340 A1
- DE 19736309 A1
- CN 102702465 A